# EUROPEAN PATENT SPECIFICATION

(11) **EP 4 475 932 B1**
(45) Date of publication and mention of the grant of the patent: **14.10.2026**
(21) Application number: 23714413.4
(22) Date of filing: 07.03.2023
(51) Int. Cl.: A61M 25/09, A61M 25/00

(54) **ADAPTIVE COIL GUIDEWIRE**
ADAPTIVER SPULENFÜHRUNGSDRAHT
FIL-GUIDE DE BOBINE ADAPTATIF

(30) Priority: 07.03.2022 US 202263317321 P
(43) Date of publication of application: 18.12.2024
(73) Proprietor: Boston Scientific Scimed, Inc., Maple Grove, MN 55311 (US)
(72) Inventor: MATTESON, Jason, Beldenville, Wisconsin 54003 (US); MACIEJ, Matthew, Rogers, Minnesota 55374 (US); OLSON, Mark, New Brighton, Minnesota 55112 (US); STENGER, Nathan, St. Paul, Minnesota 55102 (US); GESSLER, Raymond, Roberts, Wisconsin 54023 (US); DAYTON, Peter, Brookline, Massachusetts 02446 (US)
(74) Representative: Vossius & Partner Patentanwälte Rechtsanwälte mbB
(86) International application number: PCT/US2023/014684
(87) International publication number: WO 2023/172537

(56) References cited:
- US-A- 4 932 419
- US-A- 5 957 903
- US-A1- 2016 375 226
- US-B1- 6 183 420

## Description

### CROSS-REFERENCE TO RELATED APPLICATIONS

This application claims the benefit of priority of U.S. Provisional Application No. 63/317,321, filed March 7, 2022.

### TECHNICAL FIELD

The present disclosure pertains to medical devices.

### BACKGROUND

The present disclosure pertains to medical devices. More particularly, the present disclosure pertains to intracorporeal medical devices. Of the known medical devices and methods, each has certain advantages and disadvantages.

US 2016/375226 A1 relates to a guidewire which includes a wire main body possessing an elongated shape and a cylindrical body possessing a cylindrical shape. The distal portion of the wire main body is positioned in the cylindrical body. The guidewire has an inner coil disposed between the wire main body and the cylindrical body. The inner coil includes a wire rod wound in a spiral shape along the outer circumferential portion of the wire main body. There is an interval between the wire rods adjacent to each other in a longitudinal direction of the wire main body that is adjustable. The guidewire also has an operation member that changes the interval between the wire rods of the inner coil.

US 4 932 419 A relates to a flexible catheter-like guidewire which has a multi-filar, cross-wound coil body terminating in a distal ball tip element. The body has a first inner coil of multiple coil wires wound in a first helical direction and a second outer coil of multiple coil wires disposed thereabout and wound in a second opposite helical direction. The body defines an elongated lumen opening proximally. The guidewire further includes a proximal handle and, extending therefrom, a core sized and adapted to be received through the proximal opening into the body lumen. The core, by movement of the handle relative to the body, is adapted for axial movement within the body for adjustment of the flexibility of the distal region of the device. A proximal connector joins the lumen, with the core removed, to a source of fluid, and a sheath associated with the body seals the lumen for transport of fluid introduced through the proximal opening, through the lumen to be delivered radially from the lumen in the distal tip region of the device.

US 6 183 420 B1 relates to an angioplasty guidewire which includes a proximal shaft formed with an axial passage and a variable stiffness intermediate section extending axially from the tubular shaft and having a corridor aligned axially with the passage and terminating at a distal joint. The intermediate section comprises a plurality of stiffening elements. A core element is slidably disposed axially through the passage and includes a distal end projecting into the corridor and attached to the distal joint while a flexible distal tip is mounted to the end of the intermediate portion and projects axially therefrom.

US 5 957 903 A relates to a variable stiffness guidewire which has a longitudinally movable core member with a proximal end and a shapable distal end. A compressible body is slidably mounted on the core member, with one end of the compressible body secured to the end of the core member, and a tubular support member is disposed about the core member with a distal end that abuts the proximal end of the compressible body. The guidewire further comprises a tensioning means at the proximal end to move the core member longitudinally with respect to the tubular support member so as to compress and reduce the flexibility of the compressible body.

### SUMMARY

The invention is defined in the appended claims. Aspects, embodiments and examples disclosed herein which do not fall within the scope of the appended claims do not form part of the invention and are merely provided for illustrative purposes. The methods mentioned in the following do not form part of the invention and are shown for illustrative purposes only.

This disclosure provides design, material, manufacturing method, and use alternatives for medical devices. As an example, an elongate medical device is adapted to provide adjustable flexibility. The elongate medical device includes an inner coil having one or more inner coil filars extending in a first direction and at an inner coil angle, the inner coil having an outer diameter and an outer coil having one or more outer filars extending in a second direction, opposite the first direction, and an outer coil angle, the outer coil having an inner diameter, where the inner diameter of the outer coil is substantially equal to the outer diameter of the inner coil and the inner coil angle is substantially equal to the outer coil angle. A hypotube extends proximally from the inner coil and the outer coil, where a proximal end of the inner coil and a proximal end of the outer coil are secured to a distal end of the hypotube. A cable extends through the inner coil and through the hypotube, where a distal end of the cable, a distal end of the inner coil and a distal end of the outer coil are all secured together at a distal end of the elongate medical device. Applying a tensile force to the cable, relative to the hypotube, causes the inner coil and outer coil in combination to increase in stiffness.

Alternatively or additionally, the one or more inner coil filars have a wire size and the one or more outer coil filars may have the same wire size.

Alternatively or additionally, the inner coil has a number of filars and the outer coil may have the same number of filars.

Alternatively or additionally, the inner diameter of the outer coil may be within approximately five percent of the outer diameter of the inner coil.

Alternatively or additionally, the inner diameter of the outer coil may be within approximately one percent of the outer diameter of the inner coil.

Alternatively or additionally, the distal end of the inner coil, the distal end of the outer coil and the distal end of the cable may all welded together.

Alternatively or additionally, the proximal end of the inner coil and the proximal end of the outer coil may be welded together and may be welded to the distal end of the hypotube.

As another example, an elongate medical device includes a distal segment adapted to provide adjustable flexibility and a proximal segment defining an elongate shaft. The distal segment includes an inner coil having one or more inner coil filars extending in a first direction and at an inner coil angle, an outer coil having one or more outer coil filars extending in a second direction, opposite the first direction, and an outer coil angle, where the inner coil angle is substantially equal to the outer coil angle, and a cable extending through the inner coil and extending proximally therefrom, a distal end of the cable welded together with a distal end of the inner coil and a distal end of the outer coil. A distal end of the elongate shaft is welded to a proximal end of the inner coil and a proximal end of the outer coil. The distal segment is adapted to increase in stiffness when a tensile force is applied to the cable.

Alternatively or additionally, the inner coil has an outer diameter and the outer coil has an inner diameter that may be about equal to the outer diameter of the inner coil.

Alternatively or additionally, the outer diameter of the inner coil may be within approximately five percent of the inner diameter of the outer coil.

Alternatively or additionally, the outer diameter of the inner coil may be within approximately one percent of the inner diameter of the outer coil.

Alternatively or additionally, the inner coil has a number of filars and the outer coil may have the same number of filars.

Alternatively or additionally, each of the one or more inner coil filars has a wire diameter, and each of the one or more outer coil filars has the same wire diameter.

Alternatively or additionally, the elongate shaft may include a hypotube.

As another example, a guidewire includes a coil assembly including an inner coil having one or more inner coil filars extending in a first direction and an outer coil having one or more outer coil filars extending in a second direction, the inner coil having an outer diameter that is substantially equal to an inner diameter of the outer coil. A cable has an attachment point that is coupled together with a distal end of the inner coil and a distal end of the outer coil, the cable extending freely in a proximal direction from the attachment point. The cable is adapted to provide a compressive force to the coil assembly, thereby temporarily increasing a stiffness of the coil assembly.

Alternatively or additionally, the one or more inner coil filers extend at a coil angle and the one or more outer coil filers may extend at the same coil angle.

Alternatively or additionally, the outer diameter of the inner may be is within approximately five percent of the inner diameter of the outer coil.

Alternatively or additionally, the outer diameter of the inner coil may be within approximately one percent of the inner diameter of the outer coil.

Alternatively or additionally, the inner coil has a number of filars and the outer coil may have the same number of filars.

Alternatively or additionally, each of the one or more inner coil filars has a wire diameter, and each of the one or more outer coil filars may have the same wire diameter.

The above summary of some embodiments is not intended to describe each disclosed embodiment or every implementation of the present disclosure. The figures, and detailed description, which follow, more particularly exemplify these embodiments.

### BRIEF DESCRIPTION OF THE DRAWINGS

The invention may be more completely understood in consideration of the following detailed description of various embodiments of the invention in connection with the accompanying drawings, in which:
Figure 1 is a schematic side view of an illustrative elongate medical device;
Figure 2 is a side view of the illustrative elongate medical device of Figure 1 without any external polymeric layers;
Figure 3 is a cross-sectional view taken along line 3-3 of Figure 2;
Figure 3A is a schematic cross-sectional view showing another embodiment of the present disclosure;
Figure 4 is a side view of an outer coil forming a part of the illustrative elongate medical device of Figure 1; and
Figure 5 is a side view of an inner coil forming a part of the illustrative elongate medical device of Figure 1.

While the disclosure is amenable to various modifications and alternative forms, specifics thereof have been shown by way of example in the drawings and will be described in detail. It should be understood, however, that the intention is not to limit the disclosure to the particular embodiments described. On the contrary, the intention is to cover all modifications, equivalents, and alternatives falling within the scope of the disclosure.

### DESCRIPTION

For the following defined terms, these definitions shall be applied, unless a different definition is given in the claims or elsewhere in this specification.
All numeric values are herein assumed to be modified by the term "about," whether or not explicitly indicated. The term "about" or "approximately" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The drawings, which are not necessarily to scale, depict illustrative embodiments and are not intended to limit the scope of the invention.

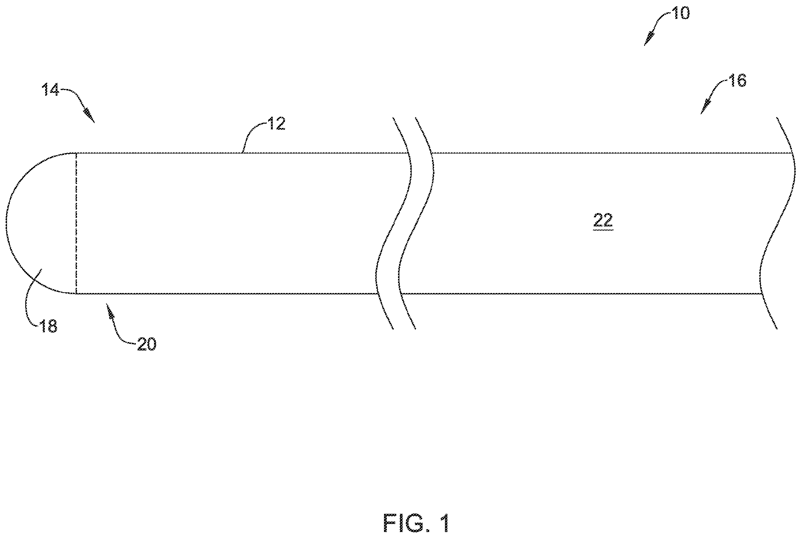

Guidewires may be used for traversing various portions of a patient's vasculature, for example. In some instances, a guidewire may be used for traversing internal structure within an organ, for example. It will be appreciated that guidewires can be subjected to conflicting performance requirements. For example, there may be a desire for a guidewire, or a portion thereof, to be highly flexible for traversing highly tortuous pathways. There may be a desire for a guidewire, or a portion thereof, to be stiff to aid in pushing the guidewire through an obstruction, for example. Torque transmission is another example of a guidewire performance characteristic. It will be appreciated that these characteristics, particularly flexibility and stiffness, are contradictory to each other. A highly flexible guidewire lacks stiffness. A stiff guidewire lacks flexibility. While some guidewires have regions that are designed to be flexible and other regions that are designed to be stiff, these characteristics are factory-set and are not adjustable. Figure 1 provides an example of a guidewire that is flexible but can be temporarily stiffened as desired.

Figure 1 is a schematic side view of an illustrative guidewire 10. The illustrative guidewire 10 is an example of an elongate medical device. The guidewire 10 includes an elongate shaft 12 that extends from a distal region 14 to a proximal region 16. In some instances, as shown, the distal region 14 may include an atraumatic tip 18 that is disposed at a distal end 20 of the elongate shaft 12. The atraumatic tip 18 may be a separate element that is secured to the distal end 20 of the elongate shaft 12 via an adhesive, if the atraumatic tip 18 is polymeric, or welding if the atraumatic tip 18 is metallic. The atraumatic tip 18 may be formed as an integral part of the elongate shaft 12, or as an integral part of one of the components forming the elongate shaft 12, for example.

In some cases, the guidewire 10 may include a polymeric layer 22 in order to provide the guidewire 10 with additional lubriciousness, for example. In some cases, the polymeric layer 22 may include a single polymeric layer. The polymeric layer 22 may include two or more different polymeric layers, depending on the exact characteristics desired for the guidewire 10. In some cases, the polymeric layer 22 may include a first polymer, mix of polymers or blend of polymers within the distal region 14 and the polymeric layer 22 may include a second polymer, mix of polymers or blend of polymers within the proximal region 16, for example. In some instances, the guidewire 10 may not include the polymeric layer 22.

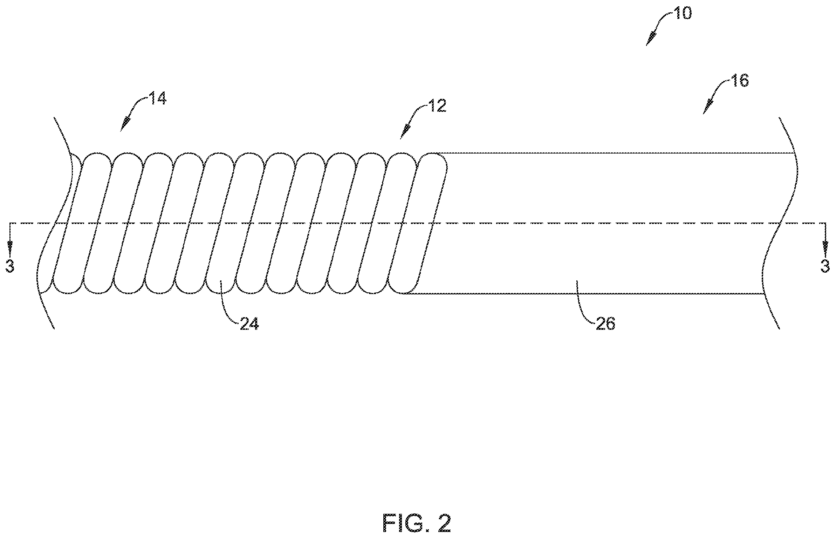

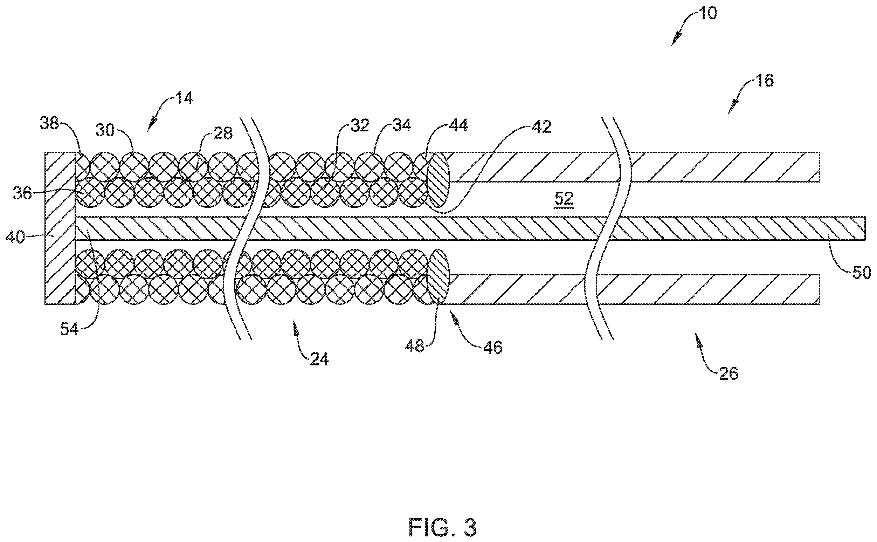

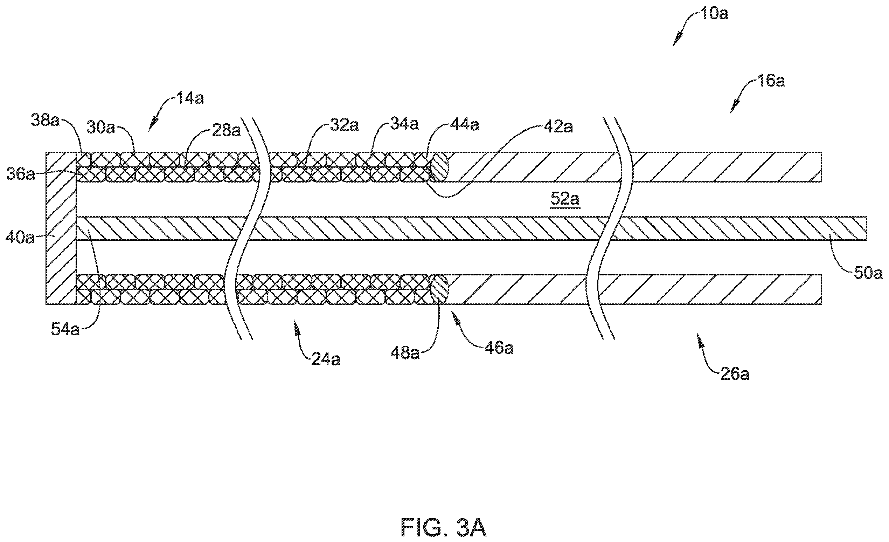

Figure 2 is a side view of the guidewire 10, without the polymeric layer 22. The guidewire 10 can be seen as including a coil segment 24 that may be considered as extending through the distal region 14 and an elongate tubular member 26 that may be considered as extending through the proximal region 16. In some instances, the coil segment 24 may include both an inner coil and an outer coil, as will be shown for example in Figure 3. In some cases, the elongate tubular member 26 may be an elongate polymeric shaft having a single layer or multiple layers. In some cases, the elongate tubular member 26 may be a hypotube. The hypotube may be metallic, for example, and in some instances may be micromachined in order to enhance flexibility of the hypotube without materially impacting pushability and torque transmission.

Figure 3 is a cross-sectional view of the guidewire 10, taken along the 3-3 line of Figure 2. As shown, the coil segment 24 includes an inner coil 28 and an outer coil 30. The inner coil 28 is formed of one or more filars 32. The outer coil 30 is formed of one or more filars 34. In some cases, the inner coil 28 has a particular number of filars 32, such as one filar 32, two filars 32, three filars 32 or more, and the outer coil 30 has the same number of filars 34. In some cases, each of the one or more filars 32 forming the inner coil 28 has a filar diameter, or filar wire size, and each of the one or more filars 34 forming the outer coil 30 has the same filar diameter, or filar wire size. The inner coil 28 and the outer coil 30 have the same number of filars 32 and 34 (respectively), and each of those filars 32 and 34 are the same size wire. The inner coil 28 and the outer coil 30 are formed of the same material.

A distal end 36 of the inner coil 28 and a distal end 38 of the outer coil 30 are secured together. As shown, the distal end 36 of the inner coil 28 and the distal end 38 of the outer coil 30 are secured together via a welding 40. A proximal end 42 of the inner coil 28 and a proximal end 44 of the outer coil 30 are secured together. As shown, the proximal end 42 of the inner coil 28 and the proximal end 44 of the outer coil 30 are welded together, and are joined to a distal end 46 of the elongate tubular member 26 via an annular welding 48.

The guidewire 10 includes a cable 50 that extends through a lumen 52 that is formed by the coil segment 24 and the elongate tubular member 26. The cable 50 has a distal end 54 that is joined together with the distal end 36 of the inner coil 28 and the distal end 38 of the outer coil 30 via the welding 40. The cable 50 may be formed of a metallic material that is suitable for being welded to the distal end 36 of the inner coil 28 and the distal end 38 of the outer coil 30. Apart from being secured at its distal end 54, the cable 50 is free to move within the lumen 52.

The coil segment 24 of the guidewire 10 provides flexibility to the distal region 14 of the guidewire 10. In some cases, the inner coil 28 and the outer coil 30 each have a filar size that is in a range of 0.025 mm to 0.127 mm (0.001 inches to 0.005 inches). The inner coil 28 and the outer coil 30 may each have a filar size that is in a range of 0.051 mm to 0.102 mm (0.002 inches to 0.004 inches), providing the coil segment 24 with flexibility. As shown, the one or more filars 32 forming the inner coil 28 and the one or more filars 34 forming the outer coil 30 are closely packed, meaning that there isn't very much space between adjoining filars. In some cases, there may be a small space between adjoining filars 32, 34, respectively.

The coil segment 24 may have any particular dimensions, depending on the desired flexibility and other desired characteristics. The coil segment 24 may have an inner diameter (ID) that is in a range of 0.508 mm to 2.032 mm (0.020 inches to 0.080 inches). It will be appreciated that the ID of the coil segment 24 corresponds to an ID of the inner coil 28. The coil segment 24 may have an outer diameter (OD) that is in a range of 0.813 mm to 0.279 mm (0.032 inches to 0.0110 inches). It will be appreciated that the OD of the coil segment 24 corresponds to an OD of the outer coil 30. In this, the ID and OD of the coil segment 24, and particular ID and OD of each of the inner coil 28 and the outer coil 30, refer to particular dimensions when the inner coil 28 and the outer coil 30 are at rest, not under the influence of any external forces that would temporarily modify the configuration of the inner coil 28 and/or the outer coil 30.

It will be appreciated that the outer diameter of the inner coil 28 has to be equal to or less than the inner diameter of the outer coil 30 in order to permit the inner coil 28 to fit within the outer coil 30. In some cases, the inner coil 28 may be temporarily constricted in diameter by winding the inner coil 28 in order to more easily fit the inner coil 28 within the outer coil 30 when the guidewire 10 is being assembled. In some cases, the outer coil 30 may be temporarily increased in diameter by unwinding the outer coil 30 in order to more easily fit the inner coil 28 within the outer coil 30. In some instances, a relative difference between the outer diameter of the inner coil 28 and the inner diameter of the outer coil 30 is such that the inner coil 28 can be extended into the outer coil 30 without requiring any temporary diameter changes.

In some cases, the outer diameter of the inner coil 28 is selected to be the same or substantially the same as the inner diameter of the outer coil 30. In this, substantially the same means that the inner diameter of the outer coil 30 is within approximately five percent of the outer diameter of the inner coil 28. In some cases, the inner diameter of the outer coil 30 is within approximately one percent of the outer diameter of the inner coil 28. The inner diameter of the outer coil 30 may be up to approximately one percent smaller, or up to approximately five percent smaller, than the outer diameter of the inner coil 28.

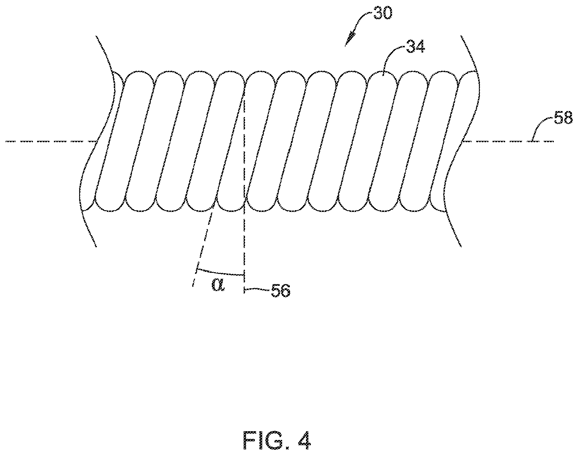

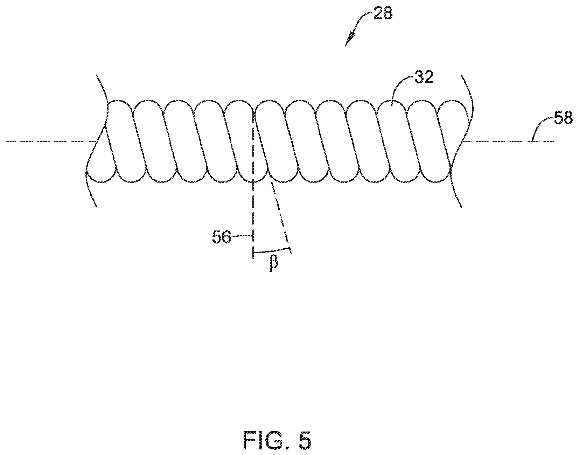

In use, the coil segment 24 is highly flexible. When the guidewire 10 encounters an obstruction or other difficult passing, it is possible to stiffen the distal region 14 of the guidewire 10 by placing the cable 50 in tension. By pulling on the cable 50, relative to the rest of the guidewire 10, the inner coil 28 and the outer coil 30 are placed in compression. Because the inner coil 28 and the outer coil 30 are essentially the same, apart from a different in winding direction (as will be discussed with respect to Figures 4 and 5), the inner coil 28 and the outer coil 30 will exert equal force in both directions. This means that the inner coil 28 and the outer coil 30 will compress down and provide increased stiffness without being forced to bend in one direction or another, or spinning.

While not shown, it is understood that the guidewire 10 may include a proximal end that allows a user to grasp the cable 50 and pull, thereby placing the cable 50 under tension when the user desires to increase the stiffness of the distal region 14 of the guidewire 10. This can allow the user to push the guidewire 10 past the obstruction, for example. Once past the obstruction, the user can release the cable 50 in order to allow the distal region 14 of the guidewire 10 to regain its original flexibility. In some cases, the user may be able to adjust how much the distal region 14 of the guidewire 10 increases in stiffness by how much of a tensile force they apply to the cable 50.

Figure 3 shows the inner coil 28 and the outer coil 30 being formed with filars 32 and 34, respectively, having a round cross-sectional shape. In some cases, the inner coil 28 and the outer coil 30 may be formed of filars having a more rectilinear cross-sectional shape, such as flat ribbon coils. Figure 3A is a schematic cross-sectional view of a guidewire 10a. As shown, a coil segment 24a includes an inner coil 28a and an outer coil 30a. The inner coil 28a is formed of one or more filars 32a. The outer coil 30a is formed of one or more filars 34a. In some cases, the inner coil 28a has a particular number of filars 32a, such as one filar 32a, two filars 32a, three filars 32a or more, and the outer coil 30a has the same number of filars 34a. In some cases, each of the one or more filars 32a forming the inner coil 28 has particular filar dimensions, and each of the one or more filars 34a forming the outer coil 30a has the same filar dimensions. The inner coil 28a and the outer coil 30a have the same number of filars 32a and 34a (respectively), and each of those filars 32a and 34a are the same size ribbon. The inner coil 28a and the outer coil 30a are formed of the same material.

A distal end 36a of the inner coil 28a and a distal end 38a of the outer coil 30a are secured together. As shown, the distal end 36a of the inner coil 28a and the distal end 38a of the outer coil 30a are secured together via a welding 40a. A proximal end 42a of the inner coil 28a and a proximal end 44a of the outer coil 30a are secured together. As shown, the proximal end 42a of the inner coil 28a and the proximal end 44a of the outer coil 30a are welded together, and are joined to a distal end 46a of an elongate tubular member 26a via an annular welding 48a.

The guidewire 10a includes a cable 50a that extends through a lumen 52a that is formed by the coil segment 24a and the elongate tubular member 26a. The cable 50a has a distal end 54a that is joined together with the distal end 36a of the inner coil 28a and the distal end 38a of the outer coil 30a via the welding 40a. The cable 50a may be formed of a metallic material that is suitable for being welded to the distal end 36a of the inner coil 28a and the distal end 38a of the outer coil 30a. Apart from being secured at its distal end 54a, the cable 50a is free to move within the lumen 52a.

The coil segment 24a of the guidewire 10a provides flexibility to the distal region 14a of the guidewire 10a. In some cases, the inner coil 28a and the outer coil 30a each have filar dimensions that are each in a range of 0.025 mm to 0.127 mm (0.001 inches to 0.005 inches). The inner coil 28a and the outer coil 30a may each have filar dimensions that are in a range of 0.051 mm to 0.102 mm (0.002 inches to 0.004 inches), providing the coil segment 24a with flexibility. In some instances, the inner coil 28a and the outer coil 30a may be formed of filars 32a and 34a, respectively, having a flat ribbon profile with a cross-sectional shape that is 0.025 mm by 0.127 mm (0.001 inches by 0.005 inches), or 0.051 mm by 0.102 mm (0.002 inches by 0.004 inches), or perhaps 0.0381 mm by 0.076 mm (0.0015 inches by 0.003 inches), or combinations thereof (0.025 mm by 0.102 mm (0.001 inches by 0.004 inches), or perhaps 0.051 mm by 0.127 mm (0.002 inches by 0.005 inches), and so on). As shown, the one or more filars 32a forming the inner coil 28a and the one or more filars 34a forming the outer coil 30a are closely packed, meaning that there isn't very much space between adjoining filars. In some cases, there may be a small space between adjoining filars 32a, 34a, respectively.

The coil segment 24a may have any particular dimensions, depending on the desired flexibility and other desired characteristics. The coil segment 24a may have an inner diameter (ID) that is in a range of 0.508 mm to 2.032 mm (0.020 inches to 0.080 inches). It will be appreciated that the ID of the coil segment 24 corresponds to an ID of the inner coil 28a. The coil segment 24a may have an outer diameter (OD) that is in a range of 0.813 mm to 0.279 mm (0.032 inches to 0.0110 inches). It will be appreciated that the OD of the coil segment 24a corresponds to an OD of the outer coil 30a. In this, the ID and OD of the coil segment 24a, and particular ID and OD of each of the inner coil 28a and the outer coil 30a, refer to particular dimensions when the inner coil 28a and the outer coil 30a are at rest, not under the influence of any external forces that would temporarily modify the configuration of the inner coil 28 and/or the outer coil 30.

It will be appreciated that the outer diameter of the inner coil 28a has to be equal to or less than the inner diameter of the outer coil 30a in order to permit the inner coil 28a to fit within the outer coil 30a. In some cases, the inner coil 28a may be temporarily constricted in diameter by winding the inner coil 28a in order to more easily fit the inner coil 28a within the outer coil 30a when the guidewire 10a is being assembled. In some cases, the outer coil 30a may be temporarily increased in diameter by unwinding the outer coil 30a in order to more easily fit the inner coil 28a within the outer coil 30a. In some instances, a relative difference between the outer diameter of the inner coil 28a and the inner diameter of the outer coil 30a is such that the inner coil 28a can be extended into the outer coil 30a without requiring any temporary diameter changes.

In some cases, the outer diameter of the inner coil 28a is selected to be the same or substantially the same as the inner diameter of the outer coil 30a. In this, substantially the same means that the inner diameter of the outer coil 30a is within approximately five percent of the outer diameter of the inner coil 28a. In some cases, the inner diameter of the outer coil 30a is within approximately one percent of the outer diameter of the inner coil 28a. The inner diameter of the outer coil 30a may be up to approximately one percent smaller, or up to approximately five percent smaller, than the outer diameter of the inner coil 28a.

In use, the coil segment 24a is highly flexible. When the guidewire 10a encounters an obstruction or other difficult passing, it is possible to stiffen the distal region 14a of the guidewire 10a by placing the cable 5a0 in tension. By pulling on the cable 50a, relative to the rest of the guidewire 10a, the inner coil 28a and the outer coil 30a are placed in compression. Because the inner coil 28a and the outer coil 30a are essentially the same, apart from a different in winding direction (as will be discussed with respect to Figures 4 and 5), the inner coil 28a and the outer coil 30a will exert equal force in both directions. This means that the inner coil 28a and the outer coil 30a will compress down and provide increased stiffness without being forced to bend in one direction or another, or spinning.

Figure 4 is a side view of a portion of the outer coil 30 while Figure 5 is a side view of a portion of the inner coil 28, showing that the outer coil 30 is wound in a first direction while the inner coil 28 is wound in a second, opposing, direction. Figure 4 shows that each of the one or more filars 34 forming the outer coil 30 are wound in a first direction in which the filars 34 slope left to right, forming an angle α (alpha) with respect to a line 56 that is orthogonal to a longitudinal axis 58 of the outer coil 30. Figure 5 shows that each of the one or more filars 32 forming the inner coil 28 are wound in a second direction in which the filars 32 slope right to left, forming an angle β (beta) with respect to the line 56 that is orthogonal to the longitudinal axis 58 of the inner coil 28.

As noted, in some cases the elongate tubular member 26 may be micromachined in order to enhance its flexibility. Accordingly, the elongate tubular member 26 may include a variety of slots (not shown) cut into the elongate tubular member 26. Slots, if present, may be disposed at the same or a similar angle with respect to a longitudinal axis of the elongate tubular member 26. Slots may be disposed at an angle that is perpendicular, or substantially perpendicular, and/or can be characterized as being disposed in a plane that is not normal to the longitudinal axis. Additionally, a group of one or more slots may be disposed at different angles relative to another group of one or more slots. The distribution and/or configuration of the slots may include, to the extent applicable, any of those disclosed in U.S. Pat. Publication No. US 2004/0181174.

Slots may be formed by methods such as micro-machining, saw-cutting (e.g., using a diamond grit embedded semiconductor dicing blade), electron discharge machining, grinding, milling, casting, molding, chemically etching or treating, or other known methods, and the like. Some example embodiments of appropriate micromachining methods and other cutting methods, and structures for tubular members including slots and medical devices including tubular members are disclosed in U.S. Pat. Publication Nos. 2003/0069522 and 2004/0181174-A2; and U.S. Pat. Nos. 6,766,720; and 6,579,246. Some example embodiments of etching processes are described in U.S. Pat. No. 5,106,455.

In at least some embodiments, slots may be formed in tubular member using a laser cutting process. The laser cutting process may include a suitable laser and/or laser cutting apparatus. For example, the laser cutting process may utilize a fiber laser. Utilizing processes like laser cutting may be desirable for a number of reasons. For example, laser cutting processes may allow a variety of different cutting patterns in a precisely controlled manner. This may include variations in the slot width, ring width, beam height and/or width, etc. Furthermore, changes to the cutting pattern can be made without the need to replace the cutting instrument (e.g., blade).

The materials that can be used for the various components of the guidewire 10 (and/or other guidewires disclosed herein) and the various tubular members disclosed herein may include those commonly associated with medical devices. The guidewire 10 and/or other components of the guidewire 10 may be made from a metal, metal alloy, polymer (some examples of which are disclosed below), a metal-polymer composite, ceramics, combinations thereof, and the like, or other suitable material. Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL^{®} 625, UNS: N06022 such as HASTELLOY^{®} C-22^{®}, UNS: N10276 such as HASTELLOY^{®} C276^{®}, other HASTELLOY^{®} alloys, and the like), nickel-copper alloys (e.g., UNS: N04400 such as MONEL^{®} 400, NICKELVAC^{®} 400, NICORROS^{®} 400, and the like), nickel-cobalt-chromium-molybdenum alloys (e.g., UNS: R30035 such as MP35-N^{®} and the like), nickel-molybdenum alloys (e.g., UNS: N10665 such as HASTELLOY^{®} ALLOY B2^{®}), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY^{®}, PHYNOX^{®}, and the like); platinum enriched stainless steel; titanium; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear that the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also can be distinguished based on its composition), which may accept only about 0.2 to 0.44 percent strain before plastically deforming.

In some embodiments, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some embodiments, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some embodiments, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Some examples of nickel titanium alloys are disclosed in U.S. Patent Nos. 5,238,004 and 6,508,803. Other suitable materials may include ULTANIUM^{™} (available from Neo-Metrics) and GUM METAL^{™} (available from Toyota). In some other embodiments, a superelastic alloy, for example a superelastic nitinol can be used to achieve desired properties.

In at least some embodiments, portions or all of the guidewire 10 may also be doped with, made of, or otherwise include a radiopaque material. Radiopaque materials are understood to be materials capable of producing a relatively bright image on a fluoroscopy screen or another imaging technique during a medical procedure. This relatively bright image aids the user of the guidewire 10 in determining its location. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of the guidewire 10 to achieve the same result.

In some embodiments, a degree of Magnetic Resonance Imaging (MRI) compatibility is imparted into the guidewire 10. For example, portions of the guidewire 10 may be made of a material that does not substantially distort the image and create substantial artifacts (e.g., gaps in the image). Certain ferromagnetic materials, for example, may not be suitable because they may create artifacts in an MRI image. Portions of the guidewire 10, may be made from a material that the MRI machine can image. Some materials that exhibit these characteristics include, for example, tungsten, cobalt-chromium-molybdenum alloys (e.g., UNS: R30003 such as ELGILOY^{®}, PHYNOX^{®}, and the like), nickel-cobalt-chromium-molybdenum alloys (e.g., UNS: R30035 such as MP35-N^{®} and the like), nitinol, and the like, and others.

Referring now to the elongate tubular member 26, the elongate tubular member 26 may be made of the same material along its length, or in some embodiments, can include portions or sections made of different materials. In some embodiments, the material used to may be chosen to impart varying flexibility and stiffness characteristics to different portions of the elongate tubular member 26. For example, a proximal section and a distal section of the elongate tubular member 26 may be formed of different materials, for example, materials having different moduli of elasticity, resulting in a difference in flexibility.

In embodiments where different portions of the elongate tubular member 26 are made of different materials, the different portions can be connected using a suitable connecting technique and/or with a connector. For example, the different portions of the elongate tubular member 26 can be connected using welding (including laser welding), soldering, brazing, adhesive, or the like, or combinations thereof. These techniques can be utilized regardless of whether or not a connector is utilized. The connector may include a structure generally suitable for connecting portions of a guidewire. One example of a suitable structure includes a structure such as a hypotube or a coiled wire which has an inside diameter sized appropriately to receive and connect to the ends of the proximal portion and the distal portion. Other suitable configurations and/or structures can be utilized for connector 26 including those connectors described in U.S. Patent Nos. 6,918,882 and 7,071,197 and/or in U.S. Patent Pub. No. 2006-0122537.

A sheath or covering (not shown) may be disposed over portions or all of the guidewire 10 and that may define a generally smooth outer surface for the guidewire 10. In other embodiments, however, such a sheath or covering may be absent from a portion of all of the guidewire 10. The sheath may be made from a polymer or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN^{®} available from DuPont), polyether block ester, polyurethane (for example, Polyurethane 85A), polypropylene (PP), polyvinylchloride (PVC), polyether-ester (for example, ARNITEL^{®} available from DSM Engineering Plastics), ether or ester based copolymers (for example, butylene/poly(alkylene ether) phthalate and/or other polyester elastomers such as HYTREL^{®} available from DuPont), polyamide (for example, DURETHAN^{®} available from Bayer or CRISTAMID^{®} available from Elf Atochem), elastomeric polyamides, block polyamide/ethers, polyether block amide (PEBA, for example available under the trade name PEBAX^{®}), ethylene vinyl acetate copolymers (EVA), silicones, polyethylene (PE), Marlex high-density polyethylene, Marlex low-density polyethylene, linear low density polyethylene (for example REXELL^{®}), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR^{®}), polysulfone, nylon, nylon-12 (such as GRILAMID^{®} available from EMS American Grilon), perfluoro(propyl vinyl ether) (PFA), ethylene vinyl alcohol, polyolefin, polystyrene, epoxy, polyvinylidene chloride (PVdC), poly(styrene-b-isobutylene-b-styrene) (for example, SIBS and/or SIBS 50A), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

In some embodiments, the exterior surface of the guidewire may be sandblasted, beadblasted, sodium bicarbonate-blasted, electropolished, etc. In these as well as in some other embodiments, a coating, for example a lubricious, a hydrophilic, a protective, or other type of coating may be applied over portions or all of the sheath, or in embodiments without a sheath over or other portions of the guidewire 10. Alternatively, the sheath may include a lubricious, hydrophilic, protective, or other type of coating. Hydrophobic coatings such as fluoropolymers provide a dry lubricity which improves guidewire handling and device exchanges. Lubricious coatings improve steerability and improve lesion crossing capability. Suitable lubricious polymers are well known in the art and may include silicone and the like, hydrophilic polymers such as high-density polyethylene (HDPE), polytetrafluoroethylene (PTFE), polyarylene oxides, polyvinylpyrrolidones, polyvinylalcohols, hydroxy alkyl cellulosics, algins, saccharides, caprolactones, and the like, and mixtures and combinations thereof. Hydrophilic polymers may be blended among themselves or with formulated amounts of water insoluble compounds (including some polymers) to yield coatings with suitable lubricity, bonding, and solubility. Some other examples of such coatings and materials and methods used to create such coatings can be found in U.S. Patent Nos. 6,139,510 and 5,772,609.

The coating and/or sheath may be formed, for example, by coating, extrusion, co-extrusion, interrupted layer co-extrusion (ILC), or fusing several segments end-to-end. The same may be true of the atraumatic tip 18. The layer may have a uniform stiffness or a gradual reduction in stiffness from the proximal end to the distal end thereof. The gradual reduction in stiffness may be continuous as by ILC or may be stepped as by fusing together separate extruded tubular segments. The outer layer may be impregnated with a radiopaque filler material to facilitate radiographic visualization. Those skilled in the art will recognize that these materials can vary widely without deviating from the scope of the present disclosure.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The invention's scope is, of course, defined in the language in which the appended claims are expressed.

## Claims

1. An elongate medical device (10), comprising:
a distal segment (24) adapted to provide adjustable flexibility, the distal segment (24) including:
an inner coil (28) having one or more inner coil filars (32) extending in a first direction and at an inner coil angle;
an outer coil (30) having one or more outer coil filars (34) extending in a second direction, opposite the first direction, and an outer coil angle, where the inner coil angle is substantially equal to the outer coil angle;
a cable (50) extending through the inner coil (28) and extending proximally therefrom, a distal end (54) of the cable (50) welded together with a distal end (36) of the inner coil (28) and a distal end (38) of the outer coil (30); and
a proximal segment defining an elongate shaft (26), a distal end (46) of the elongate shaft (26) welded to a proximal end (42) of the inner coil (28) and a proximal end (44) of the outer coil (30);
wherein the distal segment (24) is adapted to increase in stiffness when a tensile force is applied to the cable (50).

2. The elongate medical device of claim 1, wherein the inner coil (28) has an outer diameter and the outer coil (30) has an inner diameter that is about equal to the outer diameter of the inner coil (28).

3. The elongate medical device of any one of claims 1 or 2, wherein the inner coil (28) has a number of filars (32) and the outer coil (30) has the same number of filars (34).

4. The elongate medical device of any one of claims 1 to 3, wherein each of the one or more inner coil filars (32, 34) has a wire diameter, and each of the one or more outer coil filars has the same wire diameter.

5. The elongate medical device of any one of claims 1 to 4, wherein the elongate shaft (26) comprises a hypotube.

6. The elongate medical device of any of claims 1 to 5, wherein a proximal end (42) of the inner coil (28) and a proximal end (44) of the outer coil (30) are secured to a distal end (46) of the elongate shaft (26).

7. The elongate medical device of any of claims 1 to 6, wherein the cable (50) also extends through the elongate shaft (26).

8. The elongate medical device of any of claims 1 to 7, wherein a distal end (54) of the cable (50), a distal end (36) of the inner coil (28) and a distal end (38) of the outer coil (30) are all secured together at a distal end of the elongate medical device.

9. The elongate medical device of any one of claims 1 to 8, wherein the proximal end (42) of the inner coil (28) and the proximal end (44) of the outer coil (30) are welded together and are welded to the distal end (46) of the hypotube.

10. The elongate medical device of any one of claims 1 to 9, wherein the distal end (36) of the inner coil (28), the distal end (38) of the outer coil (30) and the distal end (54) of the cable (50) are all welded together.

11. The elongate medical device of any of claims 1 to 10, wherein applying a tensile force to the cable (50), relative to the elongate shaft (26), causes the inner coil (28) and outer coil (30) in combination to increase in stiffness.

12. The elongate medical device of any one of claims 1 to 11, wherein the inner diameter of the outer coil (30) is within approximately five percent or within approximately one percent of the outer diameter of the inner coil (28).

13. The elongate medical device of any one of claims 1 to 12,
wherein the configuration is such that by pulling on the cable (50) relative to the rest of the elongate medical device (10), the inner coil (28) and the outer coil (30) are placed in compression.

14. The elongate medical device of any one of claims 1 to 13,
wherein the inner coil (28) has an outer diameter, and the outer coil (30) has an inner diameter, wherein the inner diameter of the outer coil (30) is within approximately five percent of the outer diameter of the inner coil (28).

15. The elongate medical device of any one of claims 1 to 14,
wherein the elongate medical device (10) is a guidewire.

## Patentansprüche

1. Längliche medizinische Vorrichtung (10), aufweisend:
ein distales Segment (24), das geeignet ist, anpassbare Flexibilität bereitzustellen, wobei das distale Segment (24) aufweist:
eine Innenspule (28) mit einem oder mehreren Innenspulenfilamenten (32), die sich in eine erste Richtung und in einem Innenspulenwinkel erstrecken;
eine Außenspule (30) mit einem oder mehreren Außenspulenfilamenten (34), die sich in eine zur ersten Richtung entgegengesetzte zweite Richtung und in einem Außenspulenwinkel erstrecken, wobei der Innenspulenwinkel im Wesentlichen gleich dem Außenspulenwinkel ist;
ein Kabel (50), das sich durch die Innenspule (28) erstreckt und sich proximal über diese hinaus erstreckt, wobei ein distales Ende (54) des Kabels (50) mit einem distalen Ende (36) der Innenspule (28) und einem distalen Ende (38) der Außenspule (30) zusammengeschweißt ist; und
ein proximales Segment, das einen länglichen Schaft (26) definiert, wobei ein distales Ende (46) des länglichen Schafts (26) an ein proximales Ende (42) der Innenspule (28) und an ein proximales Ende (44) der Außenspule (30) geschweißt ist;
wobei das distale Segment (24) geeignet ist, an Steifheit zuzunehmen, wenn eine Zugkraft auf das Kabel (50) ausgeübt wird.

2. Längliche medizinische Vorrichtung nach Anspruch 1, wobei die Innenspule (28) einen Außendurchmesser hat und die Außenspule (30) einen Innendurchmesser hat, der ungefähr gleich dem Außendurchmesser der Innenspule (28) ist.

3. Längliche medizinische Vorrichtung nach einem der Ansprüche 1 oder 2, wobei die Innenspule (28) eine Anzahl von Filamenten (32) hat und die Außenspule (30) die gleiche Anzahl von Filamenten (34) hat.

4. Längliche medizinische Vorrichtung nach einem der Ansprüche 1 bis 3, wobei jedes des einen oder der mehreren Innenspulenfilamente (32, 34) einen Drahtdurchmesser hat und jedes des einen oder der mehreren Außenspulenfilamente den gleichen Drahtdurchmesser hat.

5. Längliche medizinische Vorrichtung nach einem der Ansprüche 1 bis 4, wobei der längliche Schaft (26) eine Hyporöhre aufweist.

6. Längliche medizinische Vorrichtung nach einem der Ansprüche 1 bis 5, wobei ein proximales Ende (42) der Innenspule (28) und ein proximales Ende (44) der Außenspule (30) an einem distalen Ende (46) des länglichen Schafts (26) befestigt sind.

7. Längliche medizinische Vorrichtung nach einem der Ansprüche 1 bis 6, wobei das Kabel (50) sich auch durch den länglichen Schaft (26) erstreckt.

8. Längliche medizinische Vorrichtung nach einem der Ansprüche 1 bis 7, wobei ein distales Ende (54) des Kabels (50), ein distales Ende (36) der Innenspule (28) und ein distales Ende (38) der Außenspule (30) alle an einem distalen Ende der länglichen medizinischen Vorrichtung miteinander verbunden sind.

9. Längliche medizinische Vorrichtung nach einem der Ansprüche 1 bis 8, wobei das proximale Ende (42) der Innenspule (28) und das proximale Ende (44) der Außenspule (30) zusammengeschweißt sind und an das distale Ende (46) der Hyporöhre geschweißt sind.

10. Längliche medizinische Vorrichtung nach einem der Ansprüche 1 bis 9, wobei das distale Ende (36) der Innenspule (28), das distale Ende (38) der Außenspule (30) und das distale Ende (54) des Kabels (50) alle zusammengeschweißt sind.

11. Längliche medizinische Vorrichtung nach einem der Ansprüche 1 bis 10, wobei Ausüben einer Zugkraft auf das Kabel (50) relativ zu dem länglichen Schaft (26) bewirkt, dass die Innenspule (28) und die Außenspule (30) in Kombination an Steifheit zunehmen.

12. Längliche medizinische Vorrichtung nach einem der Ansprüche 1 bis 11, wobei der Innendurchmesser der Außenspule (30) ungefähr fünf Prozent oder ungefähr ein Prozent des Außendurchmessers der Innenspule (28) beträgt.

13. Längliche medizinische Vorrichtung nach einem der Ansprüche 1 bis 12,
wobei die Konfiguration derart ist, dass durch Ziehen an dem Kabel (50) relativ zu dem Rest der länglichen medizinischen Vorrichtung (10) die Innenspule (28) und die Außenspule (30) in Kompression gesetzt werden.

14. Längliche medizinische Vorrichtung nach einem der Ansprüche 1 bis 13,
wobei die Innenspule (28) einen Außendurchmesser hat und die Außenspule (30) einen Innendurchmesser hat, wobei der Innendurchmesser der Außenspule (30) ungefähr fünf Prozent des Außendurchmessers der Innenspule (28) beträgt.

15. Längliche medizinische Vorrichtung nach einem der Ansprüche 1 bis 14, wobei die längliche medizinische Vorrichtung (10) ein Führungsdraht ist.

## Revendications

1. Dispositif médical allongé (10) comprenant :
un segment distal (24) adapté pour fournir la flexibilité réglable, le segment distal (24) comprenant :
une spire interne (28) ayant un ou plusieurs fils de spire interne (32) s'étendant dans une première direction et selon un angle de spire interne ;
une spire externe (30) ayant un ou plusieurs fils de spire externe (34) s'étendant dans une deuxième direction, opposée à la première direction et un angle de spire externe, où l'angle de spire interne est sensiblement égal à l'angle de spire externe ;
un câble (50) s'étendant à travers la spire interne (28) et s'étendant, de manière proximale à partir de cette dernière, une extrémité distale (54) du câble (50) étant soudée conjointement avec une extrémité distale (36) de la spire interne (28) et une extrémité distale (38) de la spire externe (30) ; et
un segment proximal définissant une tige allongée (26), une extrémité distale (46) de la tige allongée (26) étant soudée à une extrémité proximale (42) de la spire interne (28) et une extrémité proximale (44) de la spire externe (30) ;
dans lequel le segment distal (24) est adapté pour augmenter du point de vue de la rigidité, lorsqu'une force de traction est appliquée sur le câble (50).

2. Dispositif médical allongé selon la revendication 1, dans lequel la spire interne (28) a un diamètre externe et la spire externe (30) a un diamètre interne qui est à peu près égal au diamètre externe de la spire interne (28).

3. Dispositif médical allongé selon l'une quelconque des revendications 1 ou 2, dans lequel la spire interne (28) a un certain nombre de fils (32) et la spire externe (30) a le même nombre de fils (34).

4. Dispositif médical allongé selon l'une quelconque des revendications 1 à 3, dans lequel chacun des un ou plusieurs fils de spire interne (32, 34) a un diamètre de fil, et chacun des un ou plusieurs fils de spire externe a le même diamètre de fil.

5. Dispositif médical allongé selon l'une quelconque des revendications 1 à 4, dans lequel la tige allongée (26) comprend un hypotube.

6. Dispositif médical allongé selon l'une quelconque des revendications 1 à 5, dans lequel une extrémité proximale (42) de la spire interne (28) et une extrémité proximale (44) de la spire externe (30) sont fixées à une extrémité distale (46) de la tige allongée (26).

7. Dispositif médical allongé selon l'une quelconque des revendications 1 à 6, dans lequel le câble (50) s'étend également à travers la tige allongée (26).

8. Dispositif médical allongé selon l'une quelconque des revendications 1 à 7, dans lequel une extrémité distale (54) du câble (50), une extrémité distale (36) de la spire interne (28) et une extrémité distale (38) de la spire externe (30) sont toutes fixées ensemble au niveau d'une extrémité distale du dispositif médical allongé.

9. Dispositif médical allongé selon l'une quelconque des revendications 1 à 8, dans lequel l'extrémité proximale (42) de la spire interne (28) et l'extrémité proximale (44) de la spire externe (30) sont soudées ensemble et sont soudées à l'extrémité distale (46) de l'hypotube.

10. Dispositif médical allongé selon l'une quelconque des revendications 1 à 9, dans lequel l'extrémité distale (36) de la spire interne (28), l'extrémité distale (38) de la spire externe (30) et l'extrémité distale (54) du câble (50) sont toutes soudées ensemble.

11. Dispositif médical allongé selon l'une quelconque des revendications 1 à 10, dans lequel l'application d'une force de traction sur le câble (50), par rapport à la tige allongée (26), amène la spire interne (28) et la spire externe (30) en combinaison à augmenter du point de vue de la rigidité.

12. Dispositif médical allongé selon l'une quelconque des revendications 1 à 11, dans lequel le diamètre interne de la spire externe (30) est d'approximativement cinq pour cent ou d'approximativement un pour cent du diamètre externe de la spire interne (28).

13. Dispositif médical allongé selon l'une quelconque des revendications 1 à 12,
dans lequel la configuration est telle qu'en tirant sur le câble (50) par rapport au reste du dispositif médical allongé (10), la spire interne (28) et la spire externe (30) sont placées en compression.

14. Dispositif médical allongé selon l'une quelconque des revendications 1 à 13,
dans lequel la spire interne (28) a un diamètre externe, et la spire externe (30) a un diamètre interne, dans lequel le diamètre interne de la spire externe (30) est d'approximativement cinq pour cent du diamètre externe de la spire interne (28).

15. Dispositif médical allongé selon l'une quelconque des revendications 1 à 14,
dans lequel le dispositif médical allongé (10) est un fil-guide.
